# EUROPEAN PATENT SPECIFICATION

(11) **EP 2 986 334 B1**
(45) Date of publication and mention of the grant of the patent: **20.03.2019**
(21) Application number: 14716309.1
(22) Date of filing: 09.04.2014
(51) Int. Cl.: A61M 5/20, A61M 5/315, A61M 5/31

(54) **FIXATION OF A TORSION SPRING**
BEFESTIGUNG EINER TORSIONSFEDER
FIXATION D'UN RESSORT DE TORSION

(30) Priority: 18.04.2013 EP 13164216; 19.04.2013 US 201361813823 P
(43) Date of publication of application: 24.02.2016
(73) Proprietor: Novo Nordisk A/S, 2880 Bagsværd (DK)
(72) Inventor: PEDERSEN, Simon Munch, DK-København N 2200 (DK); HANSEN, Steffen, DK-Bagsværd 2880 (DK); HANSEN, Torben Strøm, DK-2880 Bagsværd (DK)
(86) International application number: PCT/EP2014/057125
(87) International publication number: WO 2014/170177

(56) References cited:
- EP-A1- 1 728 529
- WO-A2-2012/063061
- US-A- 5 104 380

## Description

### THE TECHNICAL FIELD OF THE INVENTION:

The invention relates to a torsion spring based automatic injection device for expelling settable doses of a liquid drug. The invention especially relates to securing the torsion spring in the automatic injection device and more especially to securing a helically coiled torsion spring to polymeric parts of the automatic injection device.

### DESCRIPTION OF RELATED ART:

Automatic injection devices in which a user strains a torsion spring during dose setting and wherein the torque stored in the torsion spring is utilized to expel the liquid drug has been known for decades. An early example of such automatic injection device for expelling settable dose sizes is e.g. provided in US 5,104,380.

The torsion springs used in such automatic injection devices are usually helically coiled torsion springs. The helical torsion spring is usually positioned between the housing and a rotatable dose setting member and strained by rotating the dose setting member. WO 2006/045526 discloses a helical torsion spring where the distal end has an outwardly bend for securing the torsion spring to the housing and the proximal end has an inwardly bend for attaching the torsion spring to the rotatable dose setting member.

The same is the case for WO2007/063342 in which the helical torsion spring distally is provided with a hook-like bend engaging the housing via a retaining ring moulded integrally with the housing and proximally has an inwardly bend that engages the ratchet drive shaft which is rotatable secured to the dose setting knob. Thus when a user rotates the dose setting knob the torsion spring is strained.

The helical torsion spring is further disclosed in WO 2012/063061 which discloses a number of examples on how the bended end of the helical torsion spring can be secured to parts of the injection device.

When used in an automatic injection device the torsion spring is usually build into the construction such that the axial length of the torsion spring do not change when a torque is being build up in the torsion spring as no part grows out of the injection device during dose setting. Both bended ends are secured in the injection device in axially fixed positions and rotational twisted away from each other relatively during dose setting which makes the diameter of the torsion spring decrease during dose setting.

When producing helical coiled torsion springs it is associated with additional costs to make bends at the end of the helical torsion spring. Helical torsion springs having bended end is thus more expensive than helical torsion springs without bended ends. It would thus be beneficial if a helical torsion spring without bends could be used in an automatic injection device. An example of an injection device having a torsion spring with abruptly cut ends are provided in International patent application No.: WO 2014/001318 by Novo Nordisk A/S.

When a dose is set in such injection device the abruptly cut ends of the torsion spring is rotational twisted in a direction against each other relatively which increases the diameter of the helical torsion spring. Also here are both ends secured in axially fixed positions in the injection device.

Further, if a user selects only a small dose to be injected, a certain torque needs to be available in the torsion spring in order to deliver sufficient force to expel such small dose. The force must be sufficient to overcome the friction in the dose mechanism. This requires that the torsion spring is pre-strained during manufacture of the injection device such that a certain torque is present in the torsion spring even when no dose has been selected i.e. when the dose setting mechanism is in its "zero" position. Only by having a pre-strained torsion spring is there sufficient torque to overcome the friction in the dose mechanism and expel a small dose. Mathematically, the "zero" position of the dose setting mechanism have to be positioned a certain distance up on the spring characteristic of the torsion spring such that the torsion spring already applies a certain torque in this "zero" position.

Usually both the distal end of the torsion spring and the proximal end of the torsion spring (or at least one of the ends) are secured to polymer components in the injection device as disclosed in WO 2014/001318. This however creates a problem when operating with pre-strained torsion springs because the torque loaded in the torsion spring applies stress to the polymeric parts securing the torsion spring which make the polymer creep over time. And such automatic injection devices are often stored for a substantial period of time and sometime under changing temperature condition which further exposes the polymer components under stress from the pre-tensed spring to crack propagation.

Automatic injection devices having pre-strained torsion springs therefore need to be designed such that the torque of the torsion spring is obtained by the polymeric part over a substantial area thereby reducing the stress on the polymeric parts.

### DESCRIPTION OF THE INVENTION:

It is an object of the present invention to provide an automatic torsion spring driven injection device for apportioning settable doses of a liquid drug and wherein the torsion spring has no bends at least at one end thereby reducing production costs. Further, it is an object to provide a way of mounting a torsion spring reducing stress on the polymeric parts securing the torsion spring. The reduction of stress can be understood to be the stress occurring during dose setting i.e. when straining the torsion spring or it can be the stress applied by a pre-strained torsion spring during storing of the injection device or it can be either in combination.

The invention is defined in claim 1. Accordingly in one aspect, the present invention relates to a torsion spring based automatic injection device for expelling settable doses of a liquid drug comprising a housing assembly and a dose setting assembly which is rotatable relatively to the housing assembly.
A torsion spring is encompassed between the housing assembly and the dose setting assembly such that the torsion spring is strained whenever the dose setting assembly is rotated relatively to the housing assembly.

The torsion spring is a helically coiled torsion spring having a longitudinal direction and a number of consecutive windings wherein a distal winding has a distal end and a proximal winding has a proximal end. One or both of these ends are abruptly cut to form a flat end surface. Each of the windings including the distal and the proximal winding has an outwardly pointing surface. When the injection device is pen-shaped the helically torsion spring and the injection device follows the same centre line.

At least a part of the housing assembly or a part the dose setting assembly is made from a polymeric material and comprises a spring receiving arrangement which is made from a polymeric material and comprises a first surface extending substantially parallel with the longitudinal direction of the helical torsion spring for abutting the abruptly cut flat end of the distal or the proximal end of the helical torsion spring and which spring receiving arrangement further comprises a second surface substantially parallel with the longitudinal direction of the helical torsion spring for supporting the outwardly pointing surface of the at least distal winding or the at least proximal winding.

As a result when the distal end and the proximal end of the helical torsion spring is twisted towards each other by abutment with the first surface, the outside diameter of the helical torsion spring increases and the outer surface of the torsion spring will abut with the second surface. During this abutment some of the torque built up in the helical torsion spring will be transmitted as friction against this second surface thereby releasing the first surface from some stress.

Either the distal end or the proximal end of the helical spring (or both ends) are abruptly cut to form flat end surfaces. By abruptly cut means that the wire forming the torsion spring is cut over in a direction substantially perpendicular to its length. However, the cut ends could be bended and pressed together in order to stiffen the abutment with the housing assembly and/or the dose setting assembly. The important feature being that the first surface of the spring receiving arrangement pushes on the end surface of the torsion spring when increasing the dose size.

The dose setting assembly comprises a dose setting member and the housing element comprises a housing member. One or both of these members has an integrally formed spring receiving arrangement for receiving one or both ends of the helical coiled torsion spring encompassed between the dose setting member and the housing member.

The dose setting member and the housing member is preferably arranged in a permanent axial distance and maintained in that permanent axial distance during dose setting and dose expelling thus the helical coiled torsion spring maintains its axial length during operation of the automatic injection device.

Further, the spring receiving arrangement comprises a cut-out. However, the spring receiving arrangement is not necessarily physically cut into the housing member and/or the dose setting member. The spring receiving arrangement including the cut-out is preferably formed from a polymeric material in a moulding process.

The cut out generates the first surface which one or both ends of the helical coiled torsion spring abuts.

In one embodiment a guiding surface is provided for guiding the end of the helical coiled torsion spring into abutment with the first surface. This guiding surface preferably extend in a direction substantially perpendicular to the longitudinal direction of the helical coiled torsion spring such that it intercepts the spring next to the end of the spring and lifts the end into position.

The second surface which is also in parallel with both the longitudinal direction of the helical coiled torsion spring and the first surface has in one embodiment a step-wise configuration with each step having an extension substantial equal to the diameter of the spring wire to support each winding. Each step can tilt a few degrees inwardly to provide a better grip with each windings.

### DEFINITIONS:

An **"injection pen"** is typically an injection apparatus having an oblong or elongated shape somewhat like a fountain pen for writing. Although such pens usually have a tubular cross-section, they could easily have a different cross-section such as triangular, rectangular or square or any variation around these geometries.

As used herein, the term **"drug"** is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

**"Scale drum"** is meant to be a cylinder shaped element carrying indicia indicating the size of the selected dose to the user of the injection pen. The cylinder shaped element making up the scale drum can be either solid or hollow. **"Indicia"** is meant to incorporate any kind of printing or otherwise provided symbols e.g. engraved or adhered symbols. These symbols are preferably, but not exclusively, Arabian numbers from "0" to "9". In a traditional injection pen configuration the indicia is viewable through a window provided in the housing.

**"Cartridge"** is the term used to describe the container containing the drug. Cartridges are usually made from glass but could also be moulded from any suitable polymer. A cartridge or ampoule is preferably sealed at one end by a pierceable membrane referred to as the **"septum"** which can be pierced e.g. by the back-end of a needle cannula. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container - rigid or flexible - can be used to contain the drug.

Further the term **"injection needle"** defines a piercing member adapted to penetrate the skin of a subject for the purpose of delivering or removing a liquid.

All references, including publications, patent applications, and patents, cited herein are incorporated by reference in their entirety and to the same extent as if each reference were individually and specifically indicated to be incorporated by reference and were set forth in its entirety herein.
All headings and sub-headings are used herein for convenience only and should not be constructed as limiting the invention in any way.
The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.
This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1A: show a cross sectional view of the torsion spring arrangement.
- Figure 1B: show a cut-over perspective view of the torsion spring arrangement of figure 1A.
- Figure 2A-B: show cross sectional views (180 degrees displaced) of the torsion spring attachment with the housing member.
- Figure 2C: show a cut-over perspective view of the torsion spring attachment with the housing member.
- Figure 3A-B: show cross sectional views (180 degrees displaced) of the housing member.
- Figure 3C: show a cut-over exploded view of the housing member.
- Figure 4A-B: show cross sectional views (180 degrees displaced) of the alternative torsion spring attachment with the housing member.
- Figure 4C: show a cut-over perspective view of the torsion spring attachment with the housing member.
- Figure 5A-B: show cross sectional views (180 degrees displaced) of the alternative housing member.
- Figure 5C: show a cut-over exploded view of the alternative housing member.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as there relative dimensions are intended to serve illustrative purposes only.

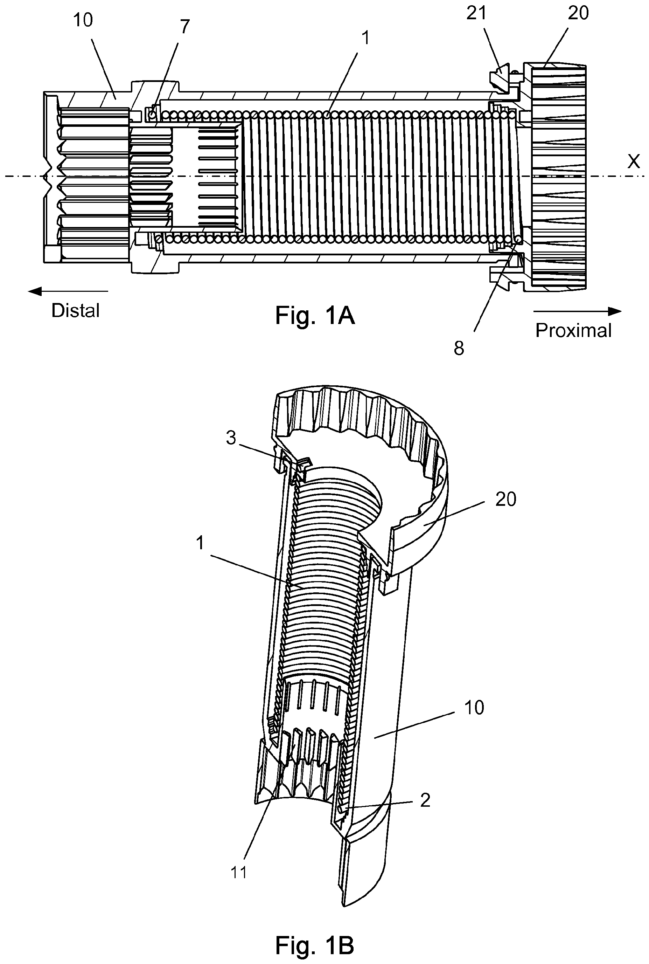

In that context it may be convenient to define that the term "distal end" in the appended figures is meant to refer to the end of the injection device which usually carries the injection needle whereas the term "proximal end" is meant to refer to the opposite end pointing away from the injection needle and usually carrying the dose dial button. The directions are indicated with arrows in figure 1A.

Figure 1A-B discloses a part of a torsion spring driven injection device according to a first embodiment of the invention. The torsion spring 1 is at its distal end 2 attached to a dose setting member 10 being a part of the dose setting assembly and at its proximal end 3 connected to a housing member 20 being part of the housing assembly.

The dose setting member 10 is further connected to a not-shown dose setting button via a toothed interface 11 such that the dose setting member 10 can be rotated when the user dials a dose. The housing member 20 is via locking protrusions 21 rotational locked to the not-shown housing but could alternatively be moulded integrally with the housing.

In WO 2014/001318 by Novo Nordisk A/S, which is incorporated by reference, the housing member (referred to as the spring base) is numbered "180" and the dose setting member (referred to as the drive tube) is numbered "170". The dose setting member"180" is connected to a distally located dose setting button (numbered "1004) via a ratchet element "185". A scale drum "160" is slidable connected to dose setting member "180". In the present invention a scale drum carrying indicia can be axially slidable connected to the dose setting member 10 which again is part of the dose setting assembly.

Whenever the user dials a dose by rotating the dose setting button, the dose setting member 10 rotates with it thereby straining the torsion spring 1 encompassed between the dose setting member 10 and the housing member 20.

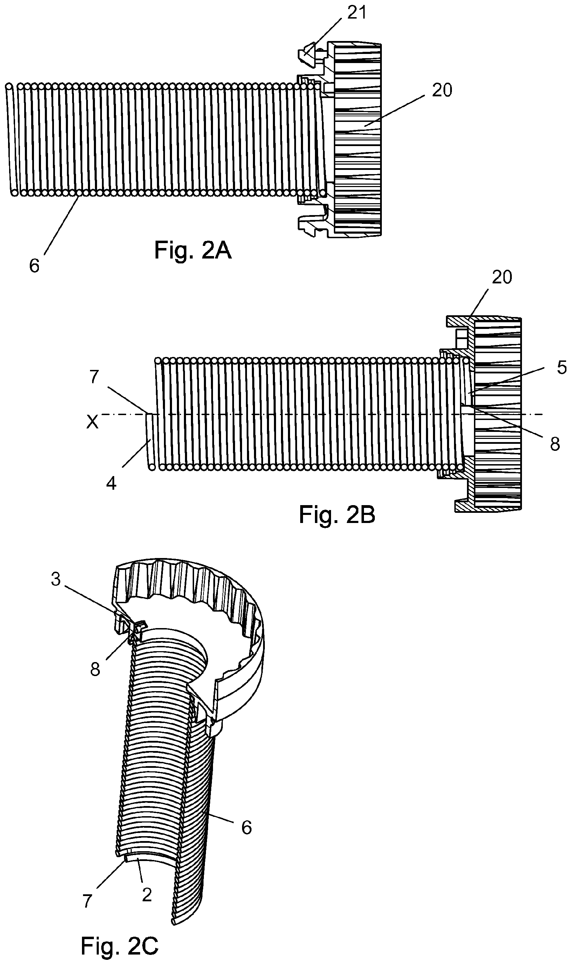

The connection between the housing member 20 and the torsion spring 1 is further disclosed in the figures 2A-C, 3A-C and 4A-C.

The torsion spring 1 is helical coiled and has a distal winding 4 ending in a distal end 2 and a proximal winding 5 ending in a proximal end 3. Both these ends 2, 3 are abruptly cut to form flat end surfaces 7, 8 which are best seen at the distal end 2 in figure 2B and 2C.

Further, as disclosed in figure 2C, each winding of the torsion spring 1 has an outer surface 6. Since the torsion spring 1 is coiled from a circular wire, the outer surface 6 runs in parallel with the longitudinal direction (X) of the helical spring 1 which is also the longitudinal direction of the injection device.

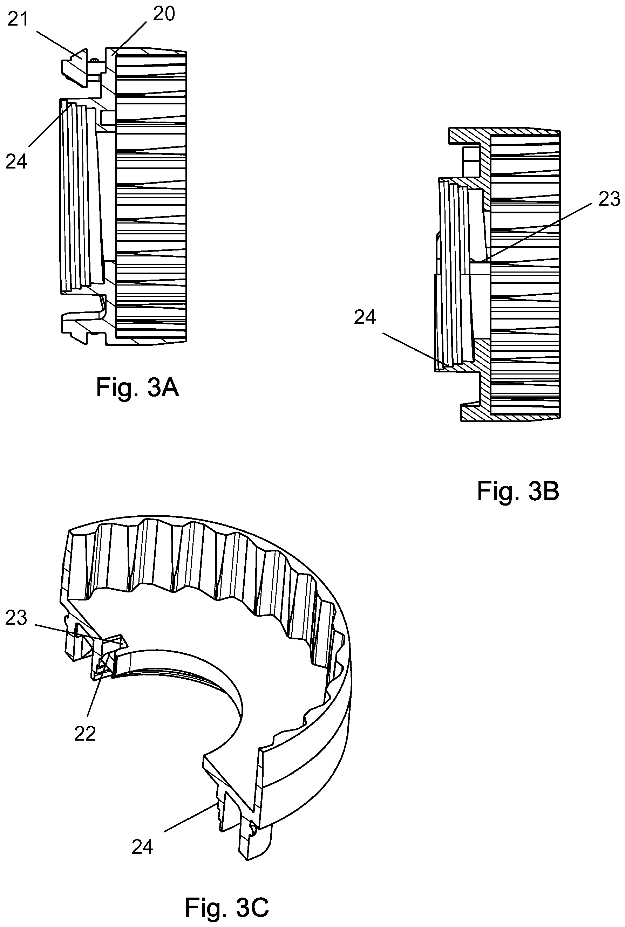

The spring receiving arrangement of the housing member 20 is further shown in the figures 3A to 5C. A similar spring receiving arrangement can be provided in the dose setting member 10 as indicated in figure 1A-1B.

The arrangement has a cut-out 22 having a first surface 23 which is parallel to the longitudinal axis X of the torsion spring 1 such that the abruptly cut proximal surface 8 of the torsion spring 1 abuts this first surface 23 when the user strains the torsion spring 1.

Distally, the housing member 20 is provided with a second surface 24 also being in parallel with the longitudinal direction X of the torsion spring 1.

When the torsion spring 1 is strained by rotating the dose setting member 10 relatively to the housing member 20, the proximal flat end surface 8 abuts the first surface 23 and further rotation of the dose setting member 10 causes the outer diameter of the torsion spring 1 to be increased.

Since the torsion spring 1 has both its flat end surfaces 7, 8 encompassed between two similar first surfaces 23 (the other surface is the not-shown first surface of the dose setting member 10) provided in the same permanent axial distance, the diameter of the torsion spring 1 will increase as the two surfaces 23 rotate relatively to each other building up torque in the torsion spring 1.

This increase of the outer diameter of the torsion spring 1 causes the outer surface 6 of at least the proximal winding 5 to abut the second surface 24 of the housing member 20.

The friction occurring between the outer surface 6 of the torsion spring 1 and the second surface 24 means that the torque build up in the torsion spring 1 during straining is distributed to the housing member 20 over a large area whereby stressing of the first surface 23 is minimized.

The second surface 24 has in one embodiment a stepwise configuration as best seen in figure 6 wherein each step is configured to abut the outer surface 6 of consecutively windings.

Each step of the second surface 24 can alternatively tilt inwardly towards the centreline X with a small angle which would provide a better grip on each consecutive winding.

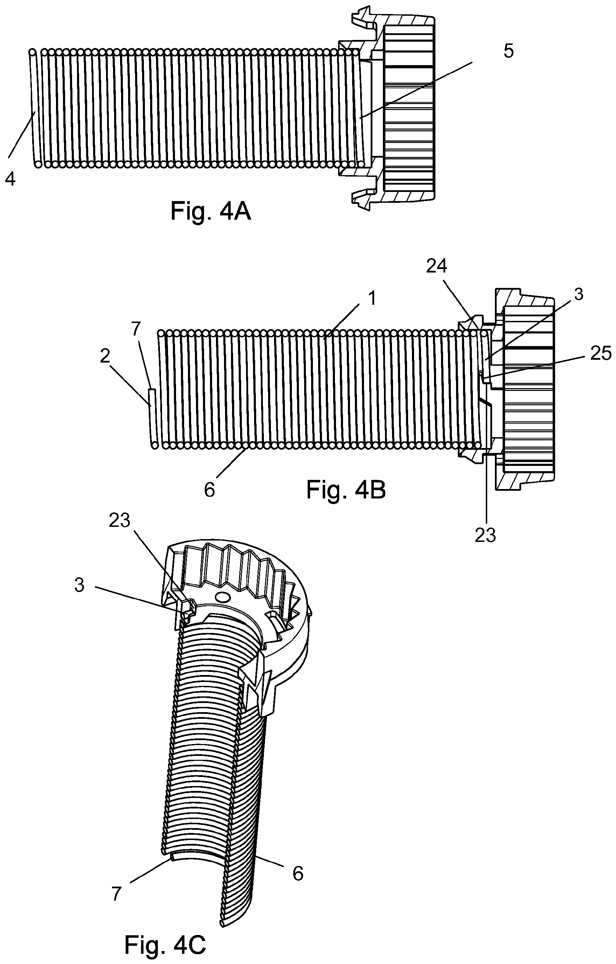

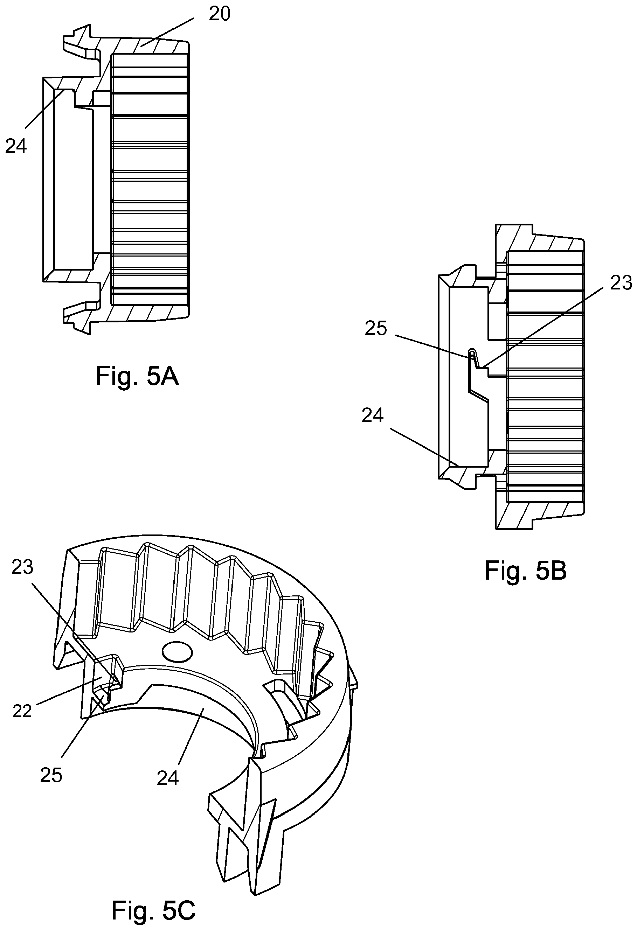

Figure 4A-C and figure 5A-C discloses an alternative embodiment wherein the second surface 24 is parallel to the longitudinal extension (X) of the helical torsion spring (1) without any steps.

Also, as best seen in figure 5B and figure 5C, the cut-out 22 is provided with a distally located guiding surface 25 for guiding the abruptly cut flat surfaces 7, 8 of the torsion spring 1 into abutment with the first surface 23.

Further, as indicated in figure 1A-1B, the dose setting member 10 can be formed in the same way such that the torsion spring 1 is fixated in the same manner both in its distal end 2 and in its proximal end 3.

Some preferred embodiments have been shown in the foregoing, but it should be stressed that the invention is not limited to these, but may be embodied in other ways within the subject matter defined in the following claims.

## Claims

1. A torsion spring based automatic injection device for expelling settable doses of a liquid drug comprising:
a housing assembly and a dose setting assembly being rotatable relatively to the housing assembly,
a torsion spring (1) encompassed between the housing assembly and the dose setting assembly such that the torsion spring (1) is strained when rotating the dose setting assembly relatively to the housing assembly, and wherein
the torsion spring (1) is helically coiled having a longitudinal direction (X) and a number of consecutive windings wherein a distal winding (4) has a distal end (2) and a proximal winding (5) has a proximal end (3), each winding further having an outwardly pointing surface (6), and wherein at least one of the housing assembly or the dose setting assembly is at least partly made from a polymeric material and comprises a spring receiving arrangement
**characterised in that**
the distal end (2) and/or the proximal end (3) of the torsion spring has no bends and is abruptly cut to form a flat end surface (7,8),
wherein the spring receiving arrangement comprises a first surface (23) substantially parallel with the longitudinal direction (X) of the helical torsion spring (1) for abutting the distal end surface (7) or the proximal end surface (8) of the helical torsion spring (1) and which spring receiving arrangement further comprises a second surface (24) substantially parallel with the longitudinal direction (X) of the helical torsion spring (1) for supporting the outwardly pointing surface (6) of the at least distal winding (4) or the at least proximal winding (5),
and wherein when the dose setting assembly are rotated relatively to the housing assembly to strain the torsion spring (1), the first surface (23) of the spring receiving arrangement is pressed against the abruptly cut end surface (7, 8) of the torsion spring (1) to strain the torsion spring (1) thereby making the outwardly pointing surface (6) of the at least distal winding (4) and/or the at least proximal winding (5) press against the second surface (24).

2. A torsion spring based automatic injection device according to claim 1, wherein the dose setting assembly comprises a polymeric dose setting member (10) in which the spring receiving arrangement is integrally formed.

3. A torsion spring based automatic injection device according to claims 1 or 2, wherein the housing assembly comprises a polymeric housing member (20) in which the spring receiving arrangement is integrally formed.

4. A torsion spring based automatic injection device according to any of the claims 1-3, wherein the spring receiving arrangement comprises a cut-out (22).

5. A torsion spring based automatic injection device according to claim 4, wherein the first surface (23) is part of the cut-out (22).

6. A torsion spring based automatic injection device according to claim 4 or 5, wherein the cut-out (22) comprises a distally located guiding surface (25) extending substantially perpendicular to the longitudinal direction (X) of the helical coiled torsion spring (1).

7. A torsion spring based automatic injection device according to any previous claim, wherein the second surface (24) comprises a step-wise configuration.

## Patentansprüche

1. Torsionsfederbasierte automatische Injektionsvorrichtung zum Ausstoßen von einstellbaren Dosen eines flüssigen Medikaments, umfassend:
eine Gehäuseanordnung und eine Dosiseinstellungsanordnung, die relativ zur Gehäuseanordnung drehbar ist,
eine Torsionsfeder (1), die zwischen der Gehäuseanordnung und der Dosiseinstellungsanordnung eingeschlossen ist, sodass die Torsionsfeder (1) gespannt wird, wenn die Dosiseinstellungsanordnung relativ zur Gehäuseanordnung gedreht wird, und wobei
die Torsionsfeder (1) spiralförmig gewunden ist und eine Längsrichtung (X) und eine Anzahl an aufeinanderfolgenden Windungen aufweist, wobei eine distale Windung (4) ein distales Ende (2) aufweist und eine proximale Windung (5) ein proximales Ende (3) aufweist und jede Windung ferner eine nach außen zeigende Fläche (6) aufweist,
und wobei mindestens eine von der Gehäuseanordnung oder der Dosiseinstellungsanordnung mindestens teilweise aus einem Polymermaterial hergestellt ist und eine Federaufnahmeanordnung umfasst,
**dadurch gekennzeichnet, dass**
das distale Ende (2) und/oder das proximale Ende (3) der Torsionsfeder keine Biegungen aufweist und abrupt geschnitten ist, sodass es eine flache Endfläche (7, 8) bildet,
wobei die Federaufnahmeanordnung umfasst
eine erste Fläche (23), die im Wesentlichen parallel zur Längsrichtung (X) der spiralförmigen Torsionsfeder (1) ist, zur Anlage an der distalen Endfläche (7) oder der proximalen Endfläche (8) der spiralförmigen Torsionsfeder (1), und wobei die Federaufnahmeanordnung ferner eine zweite Fläche (24), die im Wesentlichen parallel zur Längsrichtung (X) der spiralförmigen Torsionsfeder (1) ist, zum Unterstützen der nach außen zeigenden Fläche (6) der mindestens distalen Windung (4) oder der mindestens proximalen Windung (5) umfasst,
und wobei, wenn die Dosiseinstellungsanordnung relativ zur Gehäuseanordnung gedreht wird, um die Torsionsfeder (1) zu spannen, die erste Fläche (23) der Federaufnahmeanordnung gegen die abrupt geschnittene Endfläche (7, 8) der Torsionsfeder (1) gepresst wird, um die Torsionsfeder (1) zu spannen, wodurch die nach außen zeigende Fläche (6) der mindestens distalen Windung (4) und/oder der mindestens proximalen Windung (5) gegen die zweite Fläche (24) gepresst wird.

2. Torsionsfederbasierte automatische Injektionsvorrichtung nach Anspruch 1, wobei die Dosiseinstellungsanordnung ein Polymerdosiseinstellungselement (10) umfasst, in dem die Federaufnahmeanordnung angeformt ist.

3. Torsionsfederbasierte automatische Injektionsvorrichtung nach den Ansprüchen 1 oder 2, wobei die Gehäuseanordnung ein Polymergehäuseelement (20) umfasst, in dem die Federaufnahmeanordnung angeformt ist.

4. Torsionsfederbasierte automatische Injektionsvorrichtung nach einem der Ansprüche 1 bis 3, wobei die Federaufnahmeanordnung einen Ausschnitt (22) umfasst.

5. Torsionsfederbasierte automatische Injektionsvorrichtung nach Anspruch 4, wobei die erste Fläche (23) Teil des Ausschnitts (22) ist.

6. Torsionsfederbasierte automatische Injektionsvorrichtung nach Anspruch 4 oder 5, wobei der Ausschnitt (22) eine distal befindliche Führungsfläche (25) umfasst, die sich im Wesentlichen senkrecht zur Längsrichtung (X) der spiralförmig gewundenen Drehfeder (1) erstreckt.

7. Torsionsfederbasierte automatische Injektionsvorrichtung nach einem vorstehenden Anspruch, wobei die zweite Fläche (24) eine stufenweise Konfiguration umfasst.

## Revendications

1. Dispositif d'injection automatique à base de ressort de torsion pour l'expulsion de doses réglables d'un médicament liquide, comprenant :
un ensemble boîtier et un ensemble de réglage de dose pouvant tourner par rapport à l'ensemble boîtier,
un ressort de torsion (1) compris entre l'ensemble boîtier et l'ensemble de réglage de dose, de sorte que le ressort de torsion (1) soit sollicité lors de la rotation de l'ensemble de réglage de dose par rapport à l'ensemble boîtier, et dans lequel
le ressort de torsion (1) est enroulé hélicoïdalement avec une direction longitudinale (X) et un certain nombre d'enroulements consécutifs où un enroulement distal (4) a une extrémité distale (2) et un enroulement proximal (5) a une extrémité proximale (3), chaque enroulement ayant en outre une surface dirigée vers l'extérieur (6), et dans lequel
au moins l'un de l'ensemble boîtier ou de l'ensemble de réglage de dose est au moins partiellement fabriqué à partir d'un matériau polymère et comprend un agencement de réception de ressort
**caractérisé en ce que**
l'extrémité distale (2) et/ou l'extrémité proximale (3) du ressort de torsion sont dépourvues de courbures et sont coupées abruptement pour former une surface d'extrémité plate (7, 8),
dans lequel l'agencement de réception de ressort comprend une première surface (23) sensiblement parallèle à la direction longitudinale (X) du ressort de torsion hélicoïdal (1) pour venir en butée contre la surface d'extrémité distale (7) ou de la surface d'extrémité proximale (8) du ressort de torsion hélicoïdal (1) et ledit agencement de réception de ressort comprend en outre une seconde surface (24) sensiblement parallèle à la direction longitudinale (X) du ressort de torsion hélicoïdal (1) pour le support de la surface dirigée vers l'extérieur (6) du ou des enroulements distaux (4) ou du ou des enroulements proximaux (5),
et dans lequel, lorsque l'ensemble de réglage de dose tourne par rapport à l'ensemble boîtier pour tendre le ressort de torsion (1), la première surface (23) de l'agencement de réception de ressort exerce une pression contre la surface d'extrémité coupée abruptement (7, 8) du ressort de torsion (1) pour tendre le ressort de torsion (1) ce qui fait la surface dirigée vers l'extérieur (6) du ou des enroulements distaux (4) et/ou du ou des enroulements proximaux (5) exercer une pression contre la seconde surface (24).

2. Dispositif d'injection automatique à base de ressort de torsion selon la revendication 1, dans lequel l'ensemble de réglage de dose comprend un élément de réglage de dose polymérique (10) dans lequel l'agencement de réception de ressort est intégralement formé.

3. Dispositif d'injection automatique à base de ressort de torsion selon la revendication 1 ou 2, dans lequel l'ensemble boîtier comprend un élément de boîtier polymérique (20) dans lequel l'agencement de réception de ressort est intégralement formé.

4. Dispositif d'injection automatique à base de ressort de torsion selon l'une quelconque des revendications 1-3, dans lequel l'agencement de réception de ressort comprend une découpe (22).

5. Dispositif d'injection automatique à base de ressort de torsion selon la revendication 4, dans lequel la première surface (23) fait partie de la découpe (22).

6. Dispositif d'injection automatique à base de ressort de torsion selon la revendication 4 ou 5, dans lequel la découpe (22) comprend une surface de guidage (25) située de manière distale s'étendant sensiblement perpendiculairement à la direction longitudinale (X) du ressort de torsion hélicoïdal (1).

7. Dispositif d'injection automatique à base de ressort de torsion selon l'une quelconque des revendications précédentes, dans lequel la seconde surface (24) comprend une configuration progressive.
